(19) Europäisches Patentamt
European Patent Office
Office européen des brevets

(11) Veröffentlichungsnummer: **0 170 057**
**B1**

(12) # EUROPÄISCHE PATENTSCHRIFT

(45) Veröffentlichungstag der Patentschrift: **14.01.87**

(51) Int. Cl.[4]: **C 07 C 161/00**, C 07 D 213/76

(21) Anmeldenummer: **85107959.0**

(22) Anmeldetag: **27.06.85**

(54) **Verfahren zur Herstellung von N-Phenyl(Pyridyl)-sulfonyldiamiden.**

(30) Priorität: **30.06.84 DE 3424186**
**04.08.84 DE 3428837**

(43) Veröffentlichungstag der Anmeldung:
**05.02.86 Patentblatt 86/6**

(45) Bekanntmachung des Hinweises auf die Patenterteilung:
**14.01.87 Patentblatt 87/3**

(84) Benannte Vertragsstaaten:
**AT BE CH DE FR GB IT LI NL SE**

(56) Entgegenhaltungen:
**EP-A-0 070 467**

(73) Patentinhaber: **BASF Aktiengesellschaft, Carl-Bosch- Strasse 38, D-6700 Ludwigshafen (DE)**

(72) Erfinder: **Merkle, Hans, Dr., Brahmsstrasse 4, D-6700 Ludwigshafen (DE)**
Erfinder: **Mueller, Albrecht, Dr., Wolframstrasse 6, D-6700 Ludwigshafen (DE)**
Erfinder: **Hamprecht, Gerhard, Dr., Rote- Turm- Strasse 28, D-6940 Weinheim (DE)**
Erfinder: **Reissenweber, Gernot, Dr., Drosselstrasse 15, D-6737 Boehl- Iggelheim (DE)**

**EP 0 170 057 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die erfindung betrifft ein Verfahren zur Herstellung von N-Phenyl(Pyridyl)-Sulfonyldiamiden durch Umsetzung von 2-Amino-benzoesäure-(nicotinsäure)derivaten mit Schwefeltrioxid, Chlorsulfonsäure oder Addukten von Schwefeltrioxid an tertiäre Amine in Gegenwart eines Verdünnungsmittels und eines tertiären Amins und Umsetzung des so erhaltenen Sulfamidsäuresalzes mit einem Primären Amin und einem Fhosphorhalogenid bzw. durch Umsetzung eines 2-Amino-benzoesäure(nicotinsäure)derivats mit einer N-Alkyl(cycloalkyl)-sulfamidsäure oder eines Salzes dieser Säure in Gegenwart eines Verdünnungsmittels und eines Phosphorhalogenids.

Es ist bekannt, H-(2-Methoxycarbonyl-Phenyl)-N'-isopropyl-sulfonyldiamid durch Umsetzung von Anthranilsäuremethylester mit Chlorsulfonsäure in Gegenwart einer Pyridinbase, die auch als Verdünnungsmittel dient, und des so erhaltenen Sulfamidsäuresalzes mit Isopropylamin in Gegenwart von Phosphorpentoxid als Dehydratisierungsagens herzustellen (EP-A 0 070 467). Dieses Verfahren weist eine Reihe von Nachteilen auf, die insbesondere die Ausführung in technischem Maßstab erschweren. Bedingt durch ungünstige Mengenverhältnisse der Reaktionspartner (hohe Uberschüsse) ist die Aufarbeitung des Reaktionsgemisches apparativ aufwendig und verlustreich. Außerdem fürt die Verwendung von Phosphorpentoxid zu einem heterogenen Reaktlonsgemisch; durch Hydrolyse des Phosphorpentoxids mit dem freiwerdenden Wasser entsteht ein klumpiger Niederschlag, der zu Verstopfungen der Apparate führt und einen optimalen Reaktionsverlauf unmöglich macht.

Es wurde gefunden, daß man N-Phenyl(Pyridyl)-sulfonyldiamide der Formel

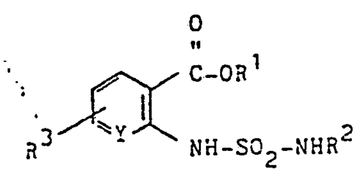

(I),

in der
$R^1$ Wasserstoff oder $C_1$-$C_5$-Alkyl,
$R^2$ $C_1$-$C_5$-Alkyl oder $C_3$-$C_8$-Cycloalkyl,
$R^3$ Wasserstoff, $C_1$-$C_{10}$-Alkyl oder $C_1$-$C_4$-Halogenalkyl und
Y CH oder N bedeuten,
ausgehend von einem 2-Amino-benzoesäure(nicotinsäure)derivat, vorteilhaft erhält, wenn man ein 2-Amino-benzoesäure(nicotinsäure)derivat der Formel

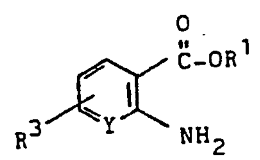

(II),

in der $R^1$, $R^3$ und Y die obengenannten Bedeutungen haben,
a) mit Schwefeltrioxid, Chlorsulfonsäure oder einem Addukt von Schwefeltrioxid an ein tertiäres Amin in Gegenwart eines tertiären Amins A und eines Verdünnungsmittels zum entsprechenden Sulfamidsäuresalz und dieses mit einem primären Amin der Formel
$R^2$-$NH_2$ (III),
in der $R^2$ die obengenannten Bedeutungen hat,
in Gegenwart eines Phosphorhalogenids als Dehydratisierungsmittel umsetzt oder
b) mit einer Sulfamidsäure der Formel
$R^2$-NH-$SO_3H$ (IV),
in der $R^2$ die obengenannten Bedeutungen hat, oder einem Salz dieser Säure in Gegenwart eines tertiären Amins A, eines Verdünnungsmittels und eines Phosphorhalogenids als Dehydratisierungsmittel umsetzt.

Im Vergleich zu dem zum Stand der Technik gehörenden Verfahren, bei dem Phosphorpentoxid als Dehydratisierungsmittel eingesetzt wird, liefern die erfindungsgemäßen Verfahren die N-Phenyl- sowie die N-Pyridyl-sulfonyldiamide in hoher Reinheit und Ausbeute. Insbesondere bei Verwendung von Phosphoroxichlorid als Dehydratisierungsagens wären eine niedrigere Ausbeute sowie Nebenreaktionen durch Phosphorsäureamidbildung und dadurch ausgelöste Folgereaktionen zu erwarten gewesen (Houben-Weyl, Methoden der organ. Chemie Bd. 12/2, S. 386, IV. Auflage, 1964).

Die Verfahrensvariante a) läßt sich durch folgendes Reaktionsschema wiedergeben:

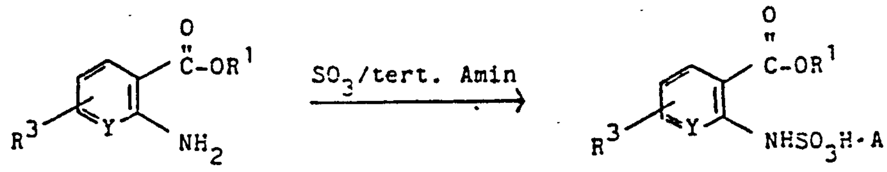


(II) (V)

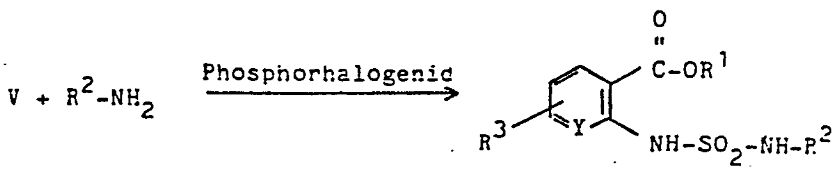


(I)

Die Verfahrensvariante b) läßt sich durch folgendes Reaktionsschema wiedergeben:

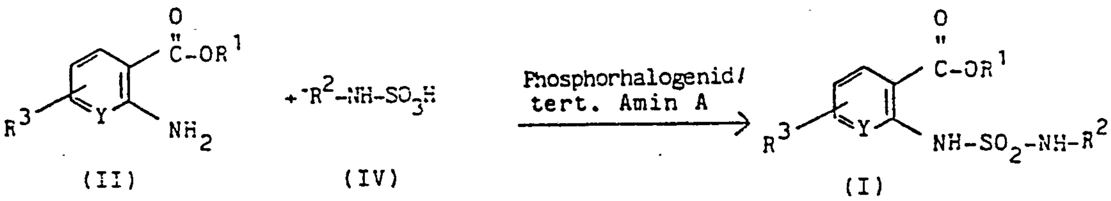


(II) (IV) (I)

Als tertiäre Amine A kommen Trialkylamine, vorzugsweise Trialkylamine mit $C_1$-$C_4$-Alkylgruppen, beispielsweise Trimethylamin, Triethylamin, Dimethylethylamin, Dimethyl-n-propylamin, Tri-n-propylamin, Triisopropylamin, Tri-n-butylamin, Triisobutylamin, Dimethyl-n-butylamin, N,N-Dialkyl-N-cycloalkyl-amine, vorzugsweise mit $C_1$-$C_4$-Alkylgruppen und $C_6$-$C_8$-Cyclo-alkylgruppen, beispielsweise N,N-Dimethyl-N-cyclohexyl-amin, N,N-Dialkylaniline, vorzugsweise mit $C_1$-$C_4$-Alkylgruppen, insbesondere Methyl oder Ethyl, beispielsweise N,N-Dimethylanilin, N,N-Diethylanilin, N-Methyl-N-ethyl-anilin, heterocyclische tertiäre Amine, wie N-Alkyl-morpholine, N-Alkyl-piperidine, N-Alkyl-imidazole, N-Alkyl-pyrrole, Alkyl-pyridine, Dialkylpyridine, vorzugsweise mit jeweils $C^1$-$C^4$-Alkylsubstituenten insbesondere Methyl oder Ethyl, beispielsweise N-Methylmorpholin, N-Ethylmorpholin, N-Methyl-piperidin, N-Ethyl-imidazol, N-Methyl-pyrrol, α-, β-, γ-Picolin, 2,4-Lutidin, 2,6-Lutidin, sowie Chinolin, Chinaldin oder Pyridin in Betracht. Auch Gemische dieser tertiären Amine können verwendet werden. Bevorzugte Amine sind Trialkylamine sowie N,N-Dialkyl-N-cycloalkylamine.

Phosphorhalogenide, die sich als Dehydratisierungsmittel eignen, sind Phosphorpentachlorid, Phosphortrichlorid und Phosphoroxichlorid, insbesondere Phosphoroxichlorid.

Geeignete Verdünnungsmittel sind sowohl polare als auch unpolare Verbindungen geeignet, beispielsweise Halogenkohlenwasserstoffe, wie Methylenchlorid, 1,1- und 1,2-Dichlorethan, 1,2-cis-Dichlorethylen, 1,1-, 1,2-, 1,3-Dichlorpropan, 1,4-Dichlorbutan, Tetrachlorkohlenstoff, Tetrachlorethan, 1,1,1-, 1,1,2-Trichlorethan, Trichlorethylen, Pentachlorethan, Trichlorfluormethan, Chlorbenzol, Dichlorbenzole, wie o-, m-, p-Dichlorbenzol, Chlortoluole, wie o-, m-, p-Chlortoluol, Dichlortoluole, wie 2,4-Dichlortoluol, Trichlorbenzole, wie 1,2,4-Trichlorbenzol, Ether, wie Ethylenglykoldimethylether, Diethylether, Di-n-propylether, Methyl-tert-butylether, Nitrokohlenwasserstoffe, wie Nitromethan, Nitrobenzol, o-, m-, p-Chlornitrebenzole, o-, p-Nitrotoluole, Kohlenwasserstoffe, wie Benzol, Toluol, Xylol, Hexan, Heptan, Octan, Tetraalkylharnstoffe, wie Tetramethylharnstoff, N,N-Dialkylamide, wie Dimethylformamid, oder Gemische aus solchen Verdünnungsmitteln. Zweckmäßigerweise verwendet man Halogenkohlenwasserstoffe oder Ether, insbesondere Dichlorethan, Chlorbenzol oder Ethylenglykoldimethylether. Die Menge an Verdünnungsmittel variiert zweckmäßigerweise zwischen 1500 bis 3000 Gewichtsteilen pro Mol Ausgangsprodukt der Formel II.

Es ist auch möglich, an Stelle eines zusätzlichen Verdünnungsmittels einen Überschuß an tertiärem Amin A als Verdünnungsmittel einzusetzen. Hierfür eignen sich vorzugsweise tertiäre Amine, wie α-Picolin, β-Picolin, γ-Picolin, 2,4-Lutidin, 2,6-Lutidin.

Außer Schwefeltrioxid und Chlorsulfonsäure eignen sich zur Herstellung der Sulfamidsäurezwischenstufe bei Verfahren a) Addukte von Schwefeltrioxid an tertiäre Amine. Dabei kommen Addukte von Schwefeltrioxid an eines der obengenannten tertiären Amine A in Betracht, vorzugsweise Addukte von Schwefeltrioxid an α-Picolin, Pyridin, Triethylamin, N,N-Dimethyl-N-cyclohexyl-amin, beispielsweise triethylamin-$SO_3$-Addukt, N,N-Dimethyl-N-cyclohexylamin-$SO_3$-Addukt. Diese Addukte lassen sich durch Zugabe von Schwefeltrioxid oder Chlorsulfonsäure zu der Lösung des tertiären Amins in einem der oben genannten Verdünnungsmittel bei einer Temperatur zwischen -20 und +50°C, vorzugsweise zwischen -10 und +30°C, herstellen. Ebenso können auch eine Lösung des Schwefeltrioxids oder der Chlorsulfonsäure vorgelegt und das tertiäre Amin in dem genannten Temperaturbereich zugegeben werden.

Die Umsetzung nach Verfahren a) wird zweckmäßigerweise so durchgeführt, daß 1 bis 2,2 Mole, vorzugsweise 1,3 bis 2 Mole, Schwefeltrioxid oder Chlorsulfonsäure nit 2 bis 6 Molen, vorzugsweise 2,5 bis 5 Molen, des tertiären Amins bei einer Temperatur im Bereich zwischen -20 und +100°C, vorzugsweise -10 und +60°C, in Gegenwart des Verdünnungsmittels zur Reaktion gebracht werden. Zu der so entstehenden Lösung bzw. Suspension des Schwefeltrioxid-Amin-Adduktes (bzw. Chlorsulfonsäure-Amin-Adduktes) werden dann bei einer temperatur im Bereich zwischen -20 und +100°C, vorzugsweise +10 und +60°C, ein Mol des 2-Amino-benzoesäure(nicotinsäure)derivates der Formel 11 in Substanz oder gelöst bzw. suspendiert in einem für die Umsetzung geeigneten Verdünnungsmittel zugegeben. Nach einigen Minuten bildet sich das Salz der entsprechenden Sulfamidsäure mit der Base, welches je nach Reaktionsbedingungen als Suspension oder als Lösung vorliegt. Ebenso kann man die Sulfamidsäure auch durch Zugabe des $SO_3$-Adduktes in Substanz zu der Suspension oder Lösung des 2-Amino-benzoesäure-(nicotinsäure)derivates in einem der obengenannten Verdünnungsmittel herstellen.

Die Kondensation des Sulfamidsäuresalzes mit dem primären Amin der Formel III unter der wasserabspaltenden Wirkung von Phosphorhalogeniden erfolgt zweckmäßigerweise bei einer Temperatur zwischen 0 und +100°C, vorzugsweise zwischen +10 bis +80°C. Die Menge an primärem Amin beträgt 1 bis 4 Mole, vorzugsweise 1,2 bis 3,8 Mole, bezogen auf 1 Mol Säurederivat der Formel II. Man läßt das Amin bei einer temperatur des Reaktionsgemisches zwischen +10 und +100°C, vorzugsweise zwischen +20 und +70°C, langsam zulaufen und gibt dann bei einer Temperatur zwischen 0 und +100°C, vorzugsweise zwischen +10 und 80°C, 0,7 bis 3 Mole, vorzugsweise 1 bis 3 Mole, insbesondere 1,3 bis 2,5 Mole, Phosphorhalogenid zu. Nach einer kurzen Reaktionszeit zwischen Raumtemperatur und Siedetemperatur wird das Reaktionsgemisch mit Wasser hydrolisiert und aufgearbeitet. Alle Umsetzungen können diskontinuierlich oder kontinuierlich, drucklos oder unter Druck betrieben werden.

Bei der Verfahrensvariante b) können die Ausgangsstoffe in stöchiometrischer Menge oder im Überschuß miteinander umgesetzt werden, vorzugsweise in einem Verhältnis von 1,1 bis 3 Mol, insbesondere 1,1 bis 2 Mole Sulfamidsäuresalz der Formel IV, bezogen auf 1 Mol Säurederivat der Formel II.

Anstelle der Sulfamidsäure der Formel IV bzw. ihrer Salze, die vorzugsweise Salze der Formel $R^2$-NH-$SO_3$H-A sind, wobei A für eines der obengenannten tertiären Amine steht, können auch Reaktionsgemische aus primären Aminen der Formel III, Schwefeltrioxid oder Chlorsulfonsäure und tertiärem Amin A eingesetzt werden. Diese Gemische enthalten pro Mol Amin der Formel III 0,5 bis 2,5 Mole, vorzugsweise 0,6 bis 1,5 Mole, Schwefeltrioxid oder Chlorsulfonsäure sowie 1 bis 5 Mole, vorzugsweise 1,5 bis 4 Mole, tertiäres Amin A. Die Bildung des Sulfamidsäuresalzes der Formel IV erfolgt in der Regel bei einer Temperatur zwischen -20 und +100°C, vorzugsweise zwischen -10 und +60°C, wobei die Umsetzung diskontinuierlich oder kontinuierlich, drucklos oder unter Druck durchgeführt werden kann. Hierbei läßt man zweckmäßigerweise das Amin der Formel III zu einer Lösung bzw. Suspension des Sulfonierungsagens in einem der oben beschriebenen Verdünnungsmittel zulaufen, man kann die Zugabe jedoch auch in umgekehrter Reihenfolge durchführen.

Es ist nicht erforderlich, die so hergestellte Sulfamidsäure bzw. ihre Salze zu isolieren, das Reaktionsgemisch wird vielmehr zweckmäßigerweise unmittelbar mit dem 2-Amino-benzoesäure(nictoinsäure)derivat der Formel II umgesetzt, so daß die Sulfamidsäure bzw. ihre Salze nur in situ gebildet werden.

Die Kondensation des Säurederivats der Formel II mit dem Sulfamidsäuresalz der Formel IV wird in der Regel bei einer Temperatur von +10 bis +100°C, vorzugsweise von +20 bis +80°C, kontinuierlich oder diskontinuierlich durchgeführt. Man läßt hierzu den Ester zu einer Lösung oder Suspension des Sulfamidsäuresalzes bei einer Temperatur zwischen +20 und +45°C zulaufen, erwärmt auf +45 bis +60°C, gibt das Phosphorhalogenid zu, erwärmt auf +60 bis 80°C und rührt 1 bis 2 Stunden nach.

Nach beendeter Umsetzung kann das N-Phenyl(Pyridyl)-sulfonyldiamid der Formel I bei Verwendung von mit Wasser mischbaren Verdünnungsmitteln durch Eindampfen zur Trockene und anschließende Behandlung des Rückstandes mit Wasser oder verdünnter Salzsäure sehr leicht aus dem Reaktionsgemisch isoliert werden. Wurde ein mit Wasser nicht mischbares Verdünnungsmittel verwendet, kann beispielsweise mit verdünnter Salzsäure oder Wasser extrahiert und, gegebenenfalls nach Chromatographie, zur Trockne eingeengt werden. Man kann jedoch auch zuerst einengen und den Rückstand dann nacheinander mit verdünnter Salzsäure und mit Wasser waschen.

Die als Ausgangsstoffe benötigten 2-Amino-benzoesäure(nicotinsäure)deriva-te der Formel II sowie die primären Amine der Formel III sind aus der Literatur bekannt bzw. sie lassen sich nach bekannten Methoden synthetisieren (Beilstein, Bd. 14, S. 318, Bd. 22, S. 542).

Die Sulfamidsäuren der Formel IV bzw. ihre Salze lassen sich außer durch Umsetzung von primären Aminen der Formel III mit Schwefeltrioxid oder Chlorsulfonsäure in Gegenwart eines tertiären Amins auch durch

Umsetzung von Alkylisocyanaten mit Schwefelsäure herstellen (Angew- Chem. 93, 151-163 (1981)).

Die nach dem erfindungsgemäßen Verfahren herstellbaren N-Phenyl(Pyridyl)- sulfonyldiamide sind wertvolle Zwischenprodukte bei der Synthese von Wirkstoffen für den Pflanzenschutz sowie von pharmazeutisch wirksamen Verbindungen. So sind N-(2-Alkoxycarbonyl-phenyl)-N'-isopropylsulfonyldiamide Vorprodukte für den herbiziden Wirkstoff 3-Isopropyl-1H-2,1,3-benzothia-diazin-(4)-3H-on-2,2-dioxid, der aus diesen Diamiden durch Cyclisierung mit Natriumalkoholat erhalten werden kann (DE-OS 23 57 063). N-(3-Alkoxy-carbonyl-pyrid-2-yl)-N'-isopropylsulfonyldiamide führen durch Umsetzung mit Natriumalkoholat zu 3-Isopropyl-1H-pyridino[3,2-e]-2,1,3-thiadiazin-4(3H)-on-2,2-dioxiden (DE-OS 24 30 353).

Die Substituenten in Formel I der erfindungsgemäß herzustellenden Sulfonyldiamide haben folgende Bedeutungen: $R^1$ steht für Wasserstoff oder unverzweigtes oder verzweigtes $C_1$-$C_5$-Alkyl, vorzugsweise für $C_1$-$C_5$-Alkyl, wie Methyl, Ethyl, n-Propyl, i-Propyl, n-Butyl, s-Butyl, n-Pentyl, Isopentyl, $R^2$ steht für unverzweigtes oder verzweigtes $C_1$-$C_5$-Alkyl oder für $C_3$-$C_8$-Cycloalkyl, wie Methyl, Ethyl, Isopropyl, n-Butyl, n-Pentyl, Cyclohexyl, Cyclooctyl, $R^3$ steht für Wasserstoff, unverzweigtes oder verzweigtes $C_1$-$C_{10}$-Alkyl, vorzugsweise $C_1$-$C_4$-Alkyl, oder unverzweigtes oder verzweigtes $C_1$-$C_4$-Halogenalkyl, vorzugsweise Halogenmethyl, wie Methyl, Ethyl, i-Propyl, n-Butyl, n-Hexyl, n-Decyl, Trifluormethyl, und Y bedeutet CH oder N. Im Falle der N-Phenyl-sulfonyldiamide steht der Substituent $R^3$ = Alkyl oder Halogenalkyl in ortho- oder meta-Stellung zur Aminfunktion am Phenylring. Im Falle der N-Pyridyl-sulfonyldiamide steht $R^3$ vorzugsweise für Wasserstoff.

Die beispielhaft genannten Bedeutungen der Substituenten $R^1$, $R^2$, $R^3$ und Y gelten auch für die Formeln II, III und IV der Ausgangsstoffe.

Die folgenden Beispiele erläutern die Durchführung des erfindungsgemäßen Verfahrens. Teile bedeuten Gewichtsteile.

**Beispiel 1**

220 Teile Chlorbenzol, 63,5 Teile N,N-Dimethyl-N-cyclohexyl-amin und 19,3 Teile Isopropylamin werden vorgelegt. Zu der Lösung tropft man bei-10°C bis 0°C in 30 Min. 23,3 teile Chlorsulfonsäure. Nach 30 Min. nachrühren, wobei die temperatur sich auf ca. 10°C einstellt, tropft man 15,1 Teile Anthranilsäuremethylester in 15 Min. zu, und rührt anschließend 30 Min. bei 50°C bis 55°C nach.

Dann wird auf Raumtemperatur abgekühltund 30,6 Teile Phosphoroxichlorid werden zugegeben. Bei 85°C bis 90°C wird 30 Min. nachgerührt. Das Reaktionsgemisch wird auf 50°C abgekühlt und bei dieser Temperatur mit 300 Teilen Wasser 15 Min. ausgerührt. Nach dem Abtrennen wird die organische Phase am Rotationsverdampfer eingeengt. Der Rückstand wird mit 1500 teilen Wasser behandelt, abfiltriert und getrocknet. Man erhält 27 Teile 99,5 %iges (HPLC) N-(2-Methoxycarbonyl-phenyl)-N'-isopropyl-sulfonyldiamid vom Fp. 106°C (98,7 % d. Th.).

**Beispiel 2**

17,5 teile Chlorsulfonsäure werden innerhalb von 30 Min. bei -10°C bis 0°C zu einer Lösung von 173 Teilen Ethylenglykoldimethylether und 141 Teilen α-Picolin zugetropft. Nach 30 Min. Rühren, wobei die Temperatur sich auf ca. 10°C einstellt, tropft man innerhalb 15 Min. 15,1 Teile Anthranilsäuremethylester zu. Die Temperatur läßt man dabei auf 25°C ansteigen. Dann tropft man in 30 Min. 19,3 Teile Isopropylamin zu, läßt die Temperatur dabei auf 30°C bis 35°C ansteigen, und rührt anschließerd 30 Minuten bei 50 bis 55°C nach.

Das Reaktionsgemisch wird auf Raumtemperatur abgekühlt und mit 23 Teilen Phosphoroxichlorid versetzt. Man rührt nun weitere 30 Minuten bei 85 bis 90°C nach, kühlt dann auf ca. 30°C ab und destilliert den Ethylengykoldimethylether am Rotationsverdampfer ab. Der erhaltene Rückstand wird in 800 Teilen 10 %iger Salzsäure und anschließend mit 1000 Teilen Wasser behandelt, abfiltriert und getrocknet. Man erhält 26,3 Teile N-(2-Methoxy-carbonyl-phenyl)-N'-isopropylsulfonyldiamid vom Gehalt 95,1 % (Fp. 104°C). Die Ausbeute beträgt 91,9 % d.Th.

**Beispiel 3**

17,5 Teile Chlorsulfonsäure werden innerhalb von 30 Min. bei -10°C bis 0°C zu einer Lösung von 173 teilen Ethylenglykoldimethylether, 93,1 Teile α-Picolin und 17,7 Teile Isopropylamin zugetropft. Nach 30 Min. Rühren, wobei sich die temperatur sich auf ca. 10°C einstellt, tropft man innerhalb 15,1 Teile Anthranilsäuremethylester in 15 Min. zu. Nun rührt man 30 Min. bei 50 bis 55°C nach. Bei Raumtemperatur werden nun 23 Teile Phosphoroxichlorid zugegeben und bei 85 bis 90°C wird nun erneut 30 Min. gerührt.

Nach dem Abkühlen auf ca. 30°C wird der Ethylenglykoldimethylether am Rotationsverdampfer abdestilliert. Der erhaltene Rückstand wird mit 800 Teilen HCl 10 %ig und anschließend mit 1000 Teilen Wasser behandelt. Nach Filtration und Trocknung erhält man 27,8 teile N-(2-Methoxycarbonyl-phenyl)-m'-isopropylsulfonyldiamid

(Fp. 101°C) mit einem Gehalt von 91,3 %. Die Ausbeute beträgt 93,3 % d.Th.

**Beispiel 4**

Zu einer Lösung aus 220 Teilen Chlorbenzol, 50,5 Teilen Triethylamin und 19,3 Teilen Isopropylamin tropft man in 30 Min. bei -10°C bis 0°C 23,3 Teile Chlorsulfonsäure. Nach 30 Min. Rühren, wobei sich die temperatur auf ca. 10°C einstellt, tropft man dann 15,1 teile Anthranilsäuremethylester in 15 Min. zu. Danach rührt man dann 30 Min. bei 50°C bis 55°C nach.

Bei Raumtemperatur werden dann 23 Teile Phosphoroxichlorid zugegeben und das Reaktionsgemisch noch 30 Min. bei 85 bis 90°C gerührt. Nach Abkühlen auf ca. 50°C werden 300 Teile Wasser zugegeben und 15 Min. ausgerührt. Nach dem Abtrennen, wird die organische Phase am Rotationsverdampfer eingeengt.

Durch Ausrühren in 1000 Teile Wasser bei 50°C erhält man 26,8 teile N-(2-Methoxycarbonyl-phenyl)-N'-isopropylsulfonyldiamid mit einem Gehalt von 96,6 % d.Th. (Fp. 103°C). Die Ausbeute beträgt 95,1 % d.Th.

**Beispiel 5**

Zu einer Lösung aus 250 Teilen 1,2-Dichlorethan, 93,1 Teilen α-Picolin und 17,7 teilen Isopropylamin tropft man in 30 Min. bei -10 bis 0°C 12 Teile Schwefeltrioxid. Nach 30 Min. Rühren, wobei sich die Temperatur auf ca. 10°C einstellt, tropft man dann 15,1 Teile Amthranilsäuremethylester in 15 Min. zu. Danach rührt man 30 Min. bei 50 bis 55°C nach. Bei Raumtemperatur werden dann 23 Teile Phosphoroxichlorid zugegeben und das Reaktionsgemisch noch 30 Min. bei 85 bis 90°C gerührt. Nach Abkühlen auf ca. 50°C werden 300 Teile Wasser zugegeben und nach 15 Min. ausgerührt. Nach dem Abtrennen, wird die organische Phase am Rotationsverdampfer eingeengt. Der Rückstand wird mit 1200 teilen Wasser behandelt, filtriert und getrocknet. Man erhält 25,4 teile 96 %iges N-(2-Methoxy-carbonyl-phenyl)-N'-isopropylsulfonyldiamid von Fp. 103°C, entsprechend einer Ausbeute von 89,6 % d.Th.

**Beispiel 6**

Zu einer Lösung aus 250 teilen 1,2-Dichlorethan, 93,1 teilen α-Picolin und 17,7 Teilen Isopropylamin tropft man in 30 Min. bei -10 bis 0°C 17,5 Teile Chlorsulfonsäure. Nach 30 Min. Rühren, wobei sich die temperatur auf ca. 10°C einstellt, tropft man nun 15,1 Teile Anthranilsäuremethylester in 15 Min. zu. Danach rührt man dann 30 Min. bei 50 bis 55°C nach. Bei Raumtemperatur werden dann 23 Teile Phosphoroxichlorid zugegeben und das Reaktionsgemisch noch 30 Min. bei 85 bis 90°C gerührt. Nach Abkühlen auf ca. 50°C werden 300 Teile Wasser zugegeben und noch 15 Min. ausgerührt. Nach dem Abtrennen, wird die organische Phase mit Natriumsulfat getrocknet und am Rotationsverdampfer eingeengt.

Der Rückstand wurde mit 1000 Teilen Wasser behandelt, filtriert und getrocknet. Man erhält 25,4 Teile 95 %iges N-(2-Methoxycarbonyl-phenyl)-N'-isopropylsulfonyldiamid von Fp. 102°C, was einer Ausbeute von 88,7 % d.Th. entspricht.

**Beispiel 7**

Zu einer Lösung aus 220 Teilen Chlorbenzol, 40,6 Teilen N,N-Dimethyl-N-cyclohexyl-amin und 7,1 Teile Isopropylamin tropft man in 30 Min. bei -10 bis 0°C 14 Teile Chlorsulfonsäure. Nach 30 Min. Rühren, wobei sich die Temperatur auf ca. 10°C einstellt, tropft man 15,1 Teile Anthranilsäuremethylester in 15 Min. zu. Danach rührt man dann 30 Min. bei 50 bis 55°C nach. Bei Raumtemperatur werden 15,3 Teile Phosphoroxichlorid zugegeben und das Reaktionsgemisch noch 30 Min. bei 85 bis 90°C gerührt. Nach Abkühlen auf ca. 50°C werden 300 Teile Wasser zugegeben und noch 15 Min. ausgerührt. Nach dem Abtrennen, wird die organische Phase mit Natriumsulfat getrocknet und eingeengt.

Der Rückstand wurde mit 1000 Teilen Wasser behandelt, filtriert und getrocknet. Man erhält 23,8 Teile 96 %iges N-(2-Methoxycarbonyl-phenyl)-N'-isopropylsulionyldiamid von Fp. 105°C. Die Ausbeute beträgt 84 % d. Th.

**Beispiel 8**

Zu einer Lösung aus 220 teilen Chlorbenzol, 93,1 Teilen α-Picolin und 19,3 teile Isopropylamin tropft man in 30 Min. bei -10 bis 0°C 17,5 teile Chlorsulfonsäure. Nach 30 Min. Rühren, wobei sich die temperatur auf ca. 10°C einstellt, tropft man 15,1 Teile Anthranilsäuremethylester in 15 Min. zu. Danach rührt man 30 Min. bei 50°C bis 55°C nach. Bei Raumtemperatur werden 23 Teile Phosphoroxichlorid zugegeben und noch 30 Min. bei 85 bis 90°C nachgerührt. Nach dem Abkühlen auf ca. 50°C werden 300 Teile Wasser zugegeben und noch 15 Min. ausgerührt. Nach dem Abtrennen wird die organische Phase mit Natriumsulfat getrocknet und anschließend eingeengt.

Der Rückstand wurde in 1500 Teilen $H_2O$ aufgenommen, filtriert und getrocknet. Man erhält 25,8 Teile 95 %iges N-(2-Methoxycarbonyl-phenyl)-N'-isopropylsulfonyldiamid von Fp. 107°C. Die Ausbeute beträg 90 % d.Th.

**Beispiel 9**

Zu einer Lösung von 110 Teile Chlorbenzol, 63,5 teile N,N-Dimethyl-N-cyclohexyl-amin und 19,3 teile Isopropylamin tropft man in 30 Min. bei -10°C bis 0°C 23,3 Teile Chlorsulfonsäure. Nach 30 Min. Rühren, wobei die Temperatur auf ca. 10°C ansteigt, tropft man 15,1 Teile Anthranilsäuremethylester in 15 Min. zu. Danach rührt man bei 50°C bis 55°C nach. Bei Raumtemperatur werden 30,6 Teile Phosphoroxichlorid zugegeben und noch 30 Min. bei 85 bis 90°C nachgerührt. Nach dem Abkühlen auf ca. 50°C werden 300 Teile Wasser zugegeben und noch 15 Min. nachgerührt. Nach dem Abtrennen wird die organische Phase mit Natriumsulfat getrocknet und eingeengt.

Durch Ausrühren in 1000 teile Wasser bei 50°C erhält man nach dem Trocknen 27,5 teile N-(2-Methoxycarbonyl-phenyl)-N'-isopropylsulfonyldiamid mit einem Gehalt von 97,9 % (Fp. 107°C). Die Ausbeute beträgt 98,9 % d.Th.

**Beispiel 10**

Zu einer Lösung aus 220 Teilen Chlorbenzol, 63,5 Teilen N,N-Dimethyl-N-cyclohexyl-amin und 19,3 Teilen Isopropylamin tropft man in 30 Min. bei 10°C 23,3 Teile Chlorsulfonsäure zu. Nach 30 Min. Rühren, wobei die Temperatur auf ca. 20°C ansteigt, tropft man 15,1 Teile Anthranilsäuremethylester in 15 Min. zu. Danach rührt man noch 30 Min. bei Raumtemperatur nach. Nun werden 30,6 Teile Phosphoroxiochlorid zugegeben und 30 Min. bei 85 bis 90°C nachgerührt. Nach dem Abkühlen auf ca. 50°C werden 300 Teile Wasser zugegeben und noch 15 Min. nachgerührt. Nach dem Abtrennen wird die organische Phase mit Natriumsulfat getrocknet und eingeengt.

Durch Ausrühren des Rückstandes in 1000 teile Wasser erhält man nach dem trocknen 27 teile N-(2-Methoxycarbonyl-phenyl)-N'-isopropylsulfonyldiamid mit einem Gehalt von 95,1 % (Fp. 107°C). Die Ausbeute beträgt 94,3 % d.Th.

**Beispiel 11**

110 Teile Chlorbenzol, 63,5 Teile N,N-Dimethyl-N-cyclohexyl-amin und 19,3 Teile Isopropylamin werden vorgelegt. Zu dieser Lösung tropft man bei 10°C 23,3 Teile Chlorsulfonsäure in 30 Min. zu. Nach 30 Min. Rühren, wobei die Temperatur auf ca. 20°C ansteigt, tropft man in 15 Min. 15,1 teile Anthranilsäuremethylester zu und rührt anschließend 30 Min. bei Raumtemperatur nach.

Nun werden 30,6 Teile Phosphoroxichlorid zugegeben und bei 85 bis 90°C 30 Min. nachgerührt. Nach dem Abkühlen auf ca. 50°C werden 300 Teile Wasser zugegeben und 15 Min. nachgerührt. Anschließend wird abgetrennt und die organische Phase mit Natriumsulfat getrocknet und eingeengt. Der Rückstand wird in 1000 Teile Wasser bei 50°C 1 Std. ausgerührt. Nach dem Absaugen und Trocknen erhält man 23,7 Teile N-(2-Methoxycarbonyl-phenyl)-N'-isopropylsulfonyldiamid vom Gehalt 95,6 % (Fp. 106°C). Die Ausbeute trägt 96,2 % d.Th.

**Beispiel 12**

Zu einer Lösung aus 220 Teilen Chlorbenzol, 38,1 Teilen N,N-Dimethyl-N-cyclohexyl-amin und 19,3 Teilen Isopropylamin gibt man bei 0°C 41,4 Teile Dimethylcyclohexylamin-$SO_3$-Addukt. Bei 10°C wird 30 Min. nachrühren. Nun werden 15,1 Teile Anthranilsäuremethylester in 15 Min. zugetropft. Das Reaktionsgemisch

wird 30 Min. bei 50°C bis 55°C nachgerührt. Nach dem Abkühlen werden 30,6 Teile Phosphoroxichlorid zugegeben und noch 30 Min. bei 85 bis 90°C nachgerührt. 300 Teile Wasser werden nun bei ca. 50°C zugegeben und 15 Min. nachgerührt.

Nach dem Abtrennen wird die organische Phase mit Natriumsulfat getrocknet und am Rotationsverdampfer eingeengt. Der erhaltene Rückstand wird bei 50°C in 1000 Teilen Wasser 1 Std. ausgerührt. Nach dem Absaugen und trocknen erhält man 26,3 Teile N-(2-Methoxycarbonyl-phenyl)-N'-isopropyl-sulfonyldiamid mit einem Gehalt von 90,9 % (Fp. 103°C). Die Ausbeute beträgt 87,9 % d.Th.

**Beispiel 13**

Zu einer Lösung aus 220 Teilen Chlorbenzol, 30,3 Teilen Triethylamin und 19,3 Teile Isopropylamin gibt man bei 0°C 36,2 Teile Triethylamin-$SO_3$-Addukt und rührt 30 Min. bei 10°C nach. Nun werden 15,1 Teile Anthranilsäuremethylester in 15 Min. zugetropft und 30 Min. bei 50 bis 55°C nachgerrührt. Nach dem Abkühlen werden 30,6 Teile Phosphoroxichlorid zugegeben und noch 30 Min. bei 85 bis 90°C nachgerührt. 300 Teile Wasser werden nun bei ca. 50°C zugegeben und 15 Min. ausgerührt. Nach dem Abtrennen wird die organische Phase mit Natriumsulfat getrocknet und eingeengt. Der erhaltene Rückstand wird bei 50°C in 1000 Teilen Wasser eine Stunde ausgerührt. Nach dem Absaugen und Trocknen erhält man 27,3 teile N-(2-Methoxy-carbonyl-phenyl)-N'-isopropylsulfonyldiamid mit einem Gehalt von 94,7 % (Fp. 108°C). Die Ausbeute beträgt 95 % d.Th.

**Beispiel 14**

Zu einer Lösung aus 173 Teilen Ethylenglykoldimethylether, 63,5 Teilen N,N-Dimethyl-cyclohexyl-amin und 19,3 Teilen Isopropylamin werden in 30 Min. bei -10 bis 0°C 23,3 Teile Chlorsulfonsäure zugetropft. Nach 30 Min. nachrühren, wobei die Temperatur auf ca. 10°C ansteigt, tropft man in 15 Min. 15,1 Teile Anthranilsäuremethylester zu und rührt weitere 30 Min. bei 50 bis 55°C nach. Dann wird auf Raumtemperatur abgekühlt und 30,6 Teile Phosphoroxichlorid werden zugegeben. Bei 85 bis 90°C wird 30 Min. nachgerührt. Der Ethylenglykoldimethylether wird unter vermindertem Druck abdestilliert und der Rückstand in 800 Teilen Wasser bei Raumtemperatur ausgerührt. Nach dem Absaugen wird der Filterrückstand noch 1 Stunde bei 50°C in 1000 Teilen Wasser ausgerührt. Nach dem Absaugen und Trocknen erhält man 26,4 Teile N-(2-Methoxycarbonyl-phenyl)-N'-isopropylsulfonyldiamid mit einem Gehalt von 97,8 % (Fp. 108°C). Die Ausbeute beträgt 94,9 % d.Th.

**Beispiel 15**

Zu einer Lösung aus 173 Teilen Ethylenglykoldimethylether, 38,1 Teilen N,N-Dimethyl-N-cyclohexylamin und 19,3 teilen Isopropylamin gibt man bei 0°C 41,4 Teile N,N-Dimethyl-N-cyclohexylamin-$SO_3$-Addukt. Das Reaktionsgemisch wird 30 Min. bei 10°C nachgerührt. Nun tropft man in 15 Min. 15,1 Teile Anthranilsäuremethylester zu und rührt bei 50 bis 55°C nach. Jetzt wird abgekühlt auf Raumtemperatur und 30,6 Teile Phosphoroxichlorid werden zugegeben. Bei 85 bis 90°C werden 30 Min. nachgerührt. Der Ethylenglykoldimethylether wird unter vermindertem Druck abdestilliert. Der Rückstand wird in 800 Teile Wasser bei Raumtemperatur ausgerührt. Nach dem Absaugen wird der Filterrückstand noch eine Stunde bei 50°C in 1000 Teile Wasser ausgerührt. Nach dem Absaugen und Trocknen erhält man 25,1 Teile N-(2-Methoxycarbonyl-phenyl)-N'-isopropylsulfonyldiamid mit einem Gehalt von 96,8 % (Fp. 107°C). Die Ausbeute beträgt 89,3 % d.Th.

**Beispiel 16**

Zu einer Lösung aus 132 Teilen n-Hexan, 38,1 Teilen N,N-Dimethyl-N-cyclohexyl-amin und 19,3 Teilen Isopropylamin gibt man bei 0°C 41,4 Teile Di-methylcyclohexylamin-$SO_3$-Addukt. Nach 30 Min. Rühren bei 10°C tropft man in 15 Min. 15,1 teile Anthranilsäuremethylester zu. Anschließend wird 30 Min. bei 50 bis 55°C nachgerührt. Nun wird abgekühlt auf Raumtemperatur und 30,6 Teile Phosphoroxichlorid werden zugegeben. Anschließend wird bei 70°C 1 Std. nachgerührt. Das n-Hexan wird unter vermindertem Druck abdestilliert, und der Rückstand anschließend mit 1000 Teilen Wasser behandelt. Nach Abfiltrieren und Trocknen erhält man 23,8 Teile N-(2-Meth-oxycarbonyl-phenyl)-N'-isopropylsulfonyldiamid mit einem Gehalt von 90,2 % (Fp. 103°C). Die Ausbeute beträgt 78,9 % d.Th.

**Beispiel 17**

25,6 Teile Chlorsulfonsäure läßt man innerhalb 15 Min. unter Rühren bei -5 bis 2°C zu einer Mischung von 55,9 Teilen α-Picolin und 100 Teilen 1,2-Dichlorethan zulaufen. Es wird 15 Min. bei 18°C und 5 Minuten bei 26°C nachgerührt. Anschließend werden 11,8 Teile Isopropylamin innerhalb 15 Min. bei einem Ansteigen der Temperatur bis auf 42°C zugegeben. Nach 15 Min. Nachrühren werden bei 35 bis 42°C innerhalb von 15 Min. 22,7 Teile Anthranilsäuremethylester zugegeben und 30 Min. bei 50 bis 55°C gerührt. Innerhalb 5 Min. werden dann 24,6 Teile Phosphoroxichlorid zugeführt und 1 Stunde bei 70 bis 75°C gerührt. Das Reaktionsgemisch wird auf 30°C abgekühlt, in 900 Teilen Eiswasser eingerührt und die abgetrennte wäßrige Phase noch einmal mit 1,2-Dichlorethan extrahiert. Der organische Extrakt wird zweimal mit 0,5 n Salzsäure und mit Wasser gewaschen und zur Trockne eingeengt. Man erhält 35 teile (= 85,7 % d.Th.) N-(2-Methoxycarbonyl-phenyl)-N'-isopropylsulfonyldiamid vom Fp. 89 bis 101°C.

**Beispiel 18**

17,6 Teile Schwefeltrioxid in 80 Teilen 1,2-Dichlorethan werden innerhalb 15 Min. zu 41 Teilen α-Picolin bei -5 bis +2°C zugegeben. Nach 15 Min. Rühren bei 6°C werden innerhalb von 15 Min. 11,8 Teile Isopropylamin zugeführt, wobei die Temperatur bis auf 35°C ansteigt. Nach 15 Min. Rühren werden bei 35°C innerhalb 15 Min. 30,2 Teile Anthranilsäuremethylester zugegeben und 30 Min. bei 50 bis 55°C gerührt. Bei 30 bis 40°C läßt man dann innerhalb 5 Min. 21,5 Teile Phosphoroxichlorid zulaufen und rührt noch eine Stunde bei 70 bis 75°C. Die Reaktionsmischung wird auf 40°C abgekühlt und in 1000 Teilen Eiswasser eingerührt. Die abgetrennte Phase wird noch zweimal mit 1,2-Dichlorethan extrahiert. Der organische Extrakt wird mit 0,5 n Salzsäure dreimal extrahiert, getrockmet und chromatographiert. Nach dem Einengen erhält man 41 Teile (75,3 % d.Th.) N-(2-Methoxycarbonyl-phenyl)-N'-isopropylsulfonyldiamid vom Fp. 96 bis 102°C.

Hiervon werden 40 Teile, gelöst in 150 Teilen Methanol, mit 53 Teilen einer 30 gew. %igen Natriummethylatlösung bei 20 bis 30°C versetzt. Das Reaktionsgemisch wird eine Stunde unter Rückfluß gerührt und dann unter vermindertem Druck eingeengt. Der Rückstand wird in Wasser aufgenommen und in 200 Teile 2 n-Salzsäure eingerührt. Nach dem Absaugen, Waschen mit Wasser und Trocknen erhält man 31,5 Teile (= 89,2 % d.Th.) 3-Isopropyl-1H-2,1,3-benzothiadiazin-(4)-3H-on-2,2-dioxid vom Fp. 127 bis 132°C.

**Beispiel 19**

15,1 Teile Anthranilsäuremethylester werden in 141 g α-Picolin vorgelegt und bei 0°C mit 21 g (0,15 mol) Isopropylsulfamidsäure versetzt und 30 Min. gerührt. Danach werden bei 30°C 10,4 g Isopropylamin zugetropft, 30 Min. nachgerührt, 53,7 g Phosphoroxichlorid zugegeben und anschließend 2 h bei 80°C gehalten. Dann gibt man 1,2 1 Wasser zu, rührt 30 Min. nach und läßt auf Raumtemperatur abkühlen. Der ausgefallene Niederschlag wird abgesaugt, mit Wasser gewaschen und getrocknet. Man erhält 23 g (84,5 %) N-(2-Methoxycarbonyl-phenyl)-N'-isopropylsulfonyldiamid vom Fp. 106 bis 107°C.

**Beispiel 20**

Zu einer Lösung aus 15,1 g Anthranilsäuremethylester, 32,6 g α-Picolin und 200 ml Dichlorethan werden bei 0°C 21 g Isopropylsulfamidsäure gegeben und 30 Min. bei Raumtemperatur gerührt. Dann gibt man 24,5 g Phosphoroxidchlorid zu und rührt 90 Min. bei 85°C. Anschließend wird zweimal mit Wasser gewaschen, die organische Phase eingeengt, der verbleibende Rückstand in Wasser aufgeschlämmt, abgesaugt und getrocknet. Man erhält 25,2 g (92,6 %) N-(2-Methoxycarbonyl-phenyl)-N'-isopropylsulfonyldiamid vom Fp. 102 bis 105°C.

**Beispiel 21**

25,45 Teile Schwefeltrioxid in 130 Teilen 1,2-Dichlorethan werden bei -3°C innerhalb 15 Min. zu 61,8 Teilen α-Picolin in 80 Teilen 1,2-Dichlor-ethan gegeben. Man rührt 15 Min. nach, wobei die Temperatur bis auf 15°C ansteigt. Anschließend werden innerhalb 15 Min. 17,2 Teile Isopropylamin zugefügt, wobei eine Temperatur von 40°C erreicht wird. Nach 15 Min. Rühren bei 35°C werden 31,35 Teile 2-Amino-micotinsäuremethylester zugegeben und 30 Minuten bei 50 bis 55°C gerührt. Bei 25 bis 35°C läßt man dann 34 Teile Phosphoroxichlorid innerhalb 4 Minuten zulaufen und rührt eine Stunde bei 70 bis 75°C nach. Die Reaktionsmischung wird auf 30°C abgekühlt und in 1000 Teile Eiswasser eingerührt. Die abgetrennte wäßrige Phase wird noch zweimal mit 1,2-Dichlorethan extrahiert. Die vereinigten organischen Auszüge werden zweimal mit 0,5 n Salzsäure ausgerührt und dann mit Wasser gewaschen. Nach dem trocknen über Magnesiumsulfat und Einengen erhält man 51,5 Teile (91,6 % d.Th.) N-(3-Methoxycarbonyl-pyrid-2-yl)-N'-isopropylsulfonyldiami d vom Fp. 98 bis 108°C.

Dieses wird in 280 Teilen Methanol unter Zugabe von 67,8 Teilen 30 %igem Natriummethylat bei 25 bis 30°C gelöst und 1 1/2 Stunden bei 65°C gerührt. Die Reaktionsmischung wird unter vermindertem Druck eingeengt, in Wasser aufgenommen und nach einmaligem Extrahieren mit Diethylether in 240 Teile 2 n Salzsäure eingerührt. Der ausgefallene Niederschlag wird abgesaugt, mit Wasser gewaschen und getrocknet. Man erhält 35,9 Teile (79 % d.Th.) 3-Isopropyl-pyrido(3,2-e)-2,1,3-thiadiazin(4)on-1,1-dioxid vom Fp. 192 bis 200°C.

**Beispiel 22**

25,45 Teile Schwefeltrioxid in 130 Teilen 1,2-Dichlorethan werden bei -5 bis 0°C innerhalb 15 Min. zu 61,8 Teilen α-Picolin in 80 Teilen 1,2-Di-chlorethan gegeben. Man rührt 15 Min. nach, wobei die Temperatur bis auf 15°C ansteigt. Anschließend werden innerhalb 15 Min. 17,2 Teile Isopropylamin zugefügt, wobei eine Temperatur von 40°C erreicht wird. Nach 15 Min. Rühren bei 35°C werden 34 teile 3-Methylanthranilsäuremethylester zugegeben und 30 Minuten bei 50 bis 55°C nachgerührt. Bei 25 bis 35°C läßt man dann 34 Teile Phosphoroxichlorid innerhalb 5 Minuten zulaufen und rührt eine Stunde bei 70 bis 75°C nach. Die Reaktionsmischung wird auf 30°C abgekühlt und in 1000 Teile Eiswasser eingerührt. Die abgetrennte wäßrige Phase wird noch zweimal mit 1,2-Dichlorethan extrahiert. Die vereinigten organischen Auszüge werden zweimal mit 0,5 n Salzsäure ausgerührt und dann mit Wasser gewaschen. Nach dem trocknen über Magmesiumsulfat und Einengen erhält man 52,5 Teile (89,1 % d.Th.) N-(2-Methoxycarbonyl-6-methyl-phenyl)-N'-isopropyl-sulfonyldiamid vom Fp. 86 bis 94°C.

**Beispiel 23**

Innerhalb 15 Minuten werden bei -2 bis 0°C 17,6 Teile Schwefeltrioxid in 85 Teilen 1,2-Dichlorethan zu einer Mischung von 51,2 Teilen α-Picolin in 45 teilen 1,2-Dichlorethan gegeben. Es wird 15 Minuten nachgerührt, wobei die Temperatur bis auf 11°C ansteigt. Dann werden innerhalb 15 Min. 27,2 Teile Amthranilsäuremethylester zugeführt, wobei eine temperatur von 30°C erreicht wird. Nach 15 Min. Rühren bei innerhalb 15 Minuten 23,6 Teile Isopropylamin zugegeben, wobei die Temperatur bis auf 50°C ansteigt. Es wird 30 Minuten bei 50 bis 55°C gerührt und dann auf 35°C abgekühlt. Innerhalb 5 Minuten gibt man dann 30,7 teile Phosphoroxychlorid hinzu und rührt eine Stunde bei 70 bis 75°C nach. Die Reaktionsmischung wird auf 30°C abgekühlt und in 1000 Teile Eiswasser eingerührt. Die abgetrennte wäßrige Phase wird noch zweimal mit 1,2-Dichlorethan extrahiert. Die vereinigten organischen Auszüge werden zweimal mit 0,5 n Salzsäure ausgerührt und dann mit Wasser gewaschen. Nach dem Trocknen über Magnesiumsulfat und Einengen erhält man 42,3 Teile (86,3 % d.Th.) N-(2-Methoxycarbonyl-phenyl)-N'-isopropylsulfonyldiamid vom Fp. 95 bis 103,C.

**Beispiel 24**

Zu einer Lösung aus 220 Teilen Chlorbenzol, 63,5 Teilen N,N-Dimethyl-N-cyclohexyl-amin und 19,3 Teilen Isopropylamin tropft man in 30 Minuten bei -10 bis 0°C 23,3 Teile Chlorsulfonsäure. Nach 30 Minuten Rühren, wobei sich die temperatur auf ca. 10°C einstellt, tropft man 16,5 Teile Anthranilsäureethylester in 15 Minuten zu. Danach rührt man 30 Minuten bei 50 bis 55°C nach. Bei 20 bis 25°C werden 30,6 Teile Phosphoroxichlorid zugegeben und anschließend noch 30 Minuten bei 85 bis 90°C nachgerührt. Nach dem Abkühlen auf ca. 50°C werden 300 Teile Wasser zugegeben und nach 15 Minuten angerührt. Nach dem Abtrennen wird die organische Phase eingeengt. Der erhaltene Rückstand wird mit 1000 Teilen Wasser bei 50°C behandelt. Nach dem Abfiltrieren und Trocknen erhält man 28,3 Teile N-(2-Ethoxycarbonyl-phenyl)-N'-isopropyl-sulfonyldiamid mit einem Gehalt von 95,9 % (Fp. 99°C). Die Ausbeute beträgt 94,9 % d.Th.

**Beispiel 25**

Zu einer Lösung aus 220 Teilen Chlorbenzol, 63,5 Teilen N,N-Dimethyl-N-cyclohexyl-amin und 19,3 Teilen Isopropylamin tropft man in 30 Minuten 23,3 Teile Chlorsulfonsäure und läßt dabei die temperatur bis auf 55°C ansteigen. Nach 30 Minuten nachrühren bei 55°C tropft man 15,1 Teile Anthranilsäuremethylester in 15 Minuten zu. Danach rührt man noch 30 Minuten bei 70°C nach. Jetzt werden 30,6 Teile Phosphoroxichlorid in 10 Minuten zugetropft, wobei die temperatur bis auf 110°C ansteigt. Bei 131°C wird 15 Minuten nachgerührt. Nach dem Abkühlen auf ca. 50°C werden 300 Teile Wasser zugegeben und 15 Minuten ausgerührt. Nach dem Abtrennen

wird die organische Phase mit Natriumsulfat getrocknet und eingeengt. Der erhaltene Rückstand wird mit 1000 teilen Wasser behandelt. Nach dem Filtrieren und Trocknen erhält man 20,4 teile N-(2-Methoxycarbonyl-phenyl)-N'-isopropyl-sulfonyldiamid mit einem Gehalt von 93,5 % (Fp. 102°C). Die Ausbeute beträgt 70 % d.Th.

**Beispiel 26**

Zu einer Lösung aus 220 Teilen Chlorbenzol, 63,5 Teilen N,N-Dimethyl-N-cyclohexylamin und 19,3 Teilen Isopropylamin tropft man in 30 Minuten bei -10°C bis 0°C 23,3 Teile Chlorsulfonsäure. Nach 30 Minuten Rühren, wobei sich die Temperatur auf ca. 10°C einstellt, tropft man 19,3 Teile Anthranilsäure-n-butylester in 15 Minuten zu. Danach rührt man 30 Minuten 25 bei 50 bis 55°C nach. Bei 20 bis 25°C werden 30,6 Teile Phosphoroxychlorid zugegeben und anschließend noch 30 Minuten bei 85 bis 90°C nachgerührt. Nach dem Abkühlen auf ca. 50°C werden 300 Teile Wasser zugegeben und noch 15 Minuten ausgerührt. Nach dem Abtrennen wird die organische Phase mit Natriumsulfat getrocknet und eingeengt. Rohausbeute 35,1 g (85,4 %ig) = 95,5 % d.Th. Der Brechungsindex einer durch präparative Dünnschichtchromatographie gewonnene Probe (farbloses Öl) ist $n_D^{25}$ = 1,5189. Der erhaltene Rückstand (braunes Öl) wird mit 1000 Teilen Wasser behandelt. Nach dem Abfiltrieren und Trocknen erhält man 29,5 Teile N-(2-m-Butoxycarbonyl-phenyl)-N'-isopropyl-sulfonyldiamid mit einem Gehalt von 91,9 % ($n_D^{25}$ = 1.5200). Die Ausbeute an N-(2-n-Butoxycarbonyl-phenyl)-N'-isopropyl-sulfonyldiamid beträgt 86,3 % d.Th.

**Beispiel 27**

Zu 140 Teilen trockenem α-Picolin werden während 30 Minuten 17,5 Teile Chlorsulfonsäure getropft. Dabei soll eine Temperatur von -5 bis +5°C aufrechterhalten werden. Man rührt 15 Minuten nach, wobei die temperatur bis 20°C ansteigen kann. Innerhalb von 10 Minuten werden dann 15,1 Teile Anthranilsäuremethylester zugegeben, wobei die temperatur bis 30°C ansteigen kann. Anschließend gibt man innerhalb einer halben Stunde 19,3 Teile Monoisopropylamin zu. Währenddessen hält man die Temperatur des Reaktionsgemisches bei 30 bis 40°C. Man rührt bei 50 bis 60°C eine Stunde lang nach, kühlt auf Raumtemperatur und gibt 23 g (0,15 Mol) Phosphoroxychlorid zu. Bei 85 bis 90°C wird eine halbe Stunde lang gerührt. Nach dem Abkühlen wird das Reaktionsgemisch mit 1 l Eiswasser verdünnt. Der Rückstand wird abgesaugt, mit kaltem Wasser gewaschen und getrocknet. Die Ausbeute an N-(2-Methoxycarbonyl-phenyl)-N'-isopropyl-sulfonyldiamid vom Fp. 103°C beträgt 25,3 Teile, entsprechend 93 % d.Th. (Reinheit 97,5 %).

**Beispiel 28**

200 Teile tetramethylharnstoff, 63,5 Teile Dimethylcyclohexylamin und 19,3 Teile Isopropylamin werden vorgelegt. Zu der Lösung tropft man dann bei -10°C bis 0°C in 30 Minuten 23,3 Teile Chlorsulfonsäure. Nach 30 Min. Nachrühren, wobei die Temperatur sich auf ca. 10°C einstellt, tropft man in 15 Minuten 15,1 Teile Anthranilsäuremethylester zu und rührt anschließend eine halbe Stunde lang bei 50 bis 55°C nach. Dann wird auf Raumtemperatur abgekühlt, und 30,6 Teile Phosphoroxichlorid werden zugegeben. Bei 85 bis 90°C wird eine halbe Stunde lang nachgerührt. Das Reaktionsgemisch wird auf Raumtemperatur abgekühlt und in 2000 Teile Eiswasser eingerührt. Nach dem Absaugen wird mit Wasser nachgewaschen und getrocknet. Man erhält 25,7 Teile N-(2-Methoxycarbonyl-phenyl)-N'-isopropyl-sulfonyl-diamid mit einem Gehalt von 96,3 % (Fp. 104°C). Die Ausbeute beträgt 91 % d.th.

**Beispiel 29**

220 Teile Chlorbenzol, 93,1 Teile β-Picolin und 19,3 Teile Isopropylamin werden vorgelegt. Zu der Lösung tropft man bei -10°C bis 0°C in 30 Minuten 23,3 Teile Chlorsulfonsäure. Nach 30 Minuten Nachrühren, wobei sich die Temperatur auf ca. 10°C einstellt, tropft man in 15 Minuten 15,1 Teile Anthranilsäuremethylester zu und rührt anschließend eine halbe Stunde bei 50 bis 55°C nach. Nach dem Abkühlen auf Raumtemperatur werden 30,6 Teile Phosphoroxichlorid zugegeben. Dann wird bei 85 bis 90°C eine halbe Stunde lang nachgerührt. Das Reaktionsgemisch wird auf ca. 50°C abgekühlt und bei dieser Temperatur 15 Minuten lang mit 300 Teilen Wasser ausgerührt. Nach dem Abtrennen wird die organische Phase am Rotationsverdampfer eingeengt. Der Rückstand wird in 1000 teilen Wasser bei 50°C ausgerührt. Nach dem Absaugen und Trocknen erhält man 32,6 Teile N-(2-Methoxycarbonyl-phenyl)-N'-isopropyl-sulfonyldiamid mit einem Gehalt von 62,8 % (Fp. 86°C). Die Ausbeute beträgt 75,3 % d.th.

## Patentansprüche

1. Verfahren zur Herstellung von N-Phenyl(Pyridyl)-sulfonyldiamiden der Formel

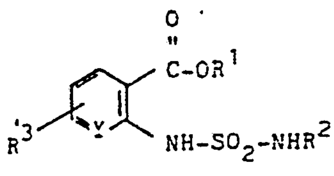

(I),

in der
$R^1$ Wasserstoff oder $C_1$-$C_5$-Alkyl,
$R^2$ $C_1$-$C_5$-Alkyl oder $C_3$-$C_8$-Cycloalkyl,
$R^3$ Wasserstoff, $C_1$-$C_{10}$-Alkyl oder $C_1$-$C_4$-Halogenalkyl und
Y CH oder N bedeuten,
dadurch gekennzeichnet, daß man ein 2-Amino-benzoesäure(nicotinsäure)-derivat der Formel

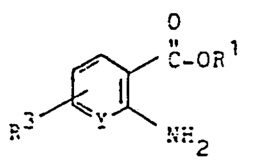

(II),

in der
$R^1$, $R^3$ und Y die obengenannten Bedeutungen haben,
a) mit Schwefeltrioxid, Chlorsulfonsäure oder einem Addukt von Schwefeltrioxid an ein tertiäres Amin in Gegenwart eines tertiären Amins A und eines Verdünnungsmittels zum entsprechenden Sulfamidsäuresalz und dieses mit einem primären Amin der Formel
$R^2NH_2$ (III),
in der
$R^2$ die obengenannten Bedeutungen hat,
in Gegenwart eines Phosphorhalogenids als Dehydratisierungsmittel umsetzt oder
b) mit einer Sulfamidsäure der Formel
$R^2$-NH-$SO_3H$ (IV)
in der
$R^2$ die obengenannten Bedeutungen hat, oder einem Salz dieser Säure in Gegenwart eines tertiären Amins A, eines Verdünngsmittels und eines Phosphorhalogenids als Dehydratisierungsmittel umsetzt.

2. Verfahren nach Anspruch 1, dadurch gekennzeichnet, daß man ein Sulfamidsäuresalz der Formels
$R^2$-NH-$SO^3H$.A,
wobei $R^2$ und A die in Anspruch 1 genannten Bedeutungen haben, verwendet.

3. Verfahren nach Anspruch 1, dadurch gekennzeichnet, daß man eine Sulfamidsäure der Formel IV oder ein Salz dieser Säure verwendet, das durch Umsetzung eines primären Amins der Formel III mit Schwefeltrioxid oder Chlorsulfonsäure in Gegenwart eines tertiären Amins A und eines Verdünnungsmittels erhalten wurde.

4. Verfahren nach Anspruch 1, dadurch gekennzeichnet, daß das Phosphorhalogenid Phosphoroxichlorid ist.

## Claims

1. A process for the preparation of N-phenyl(pyridyl)-sulfonyldiamides of the formula

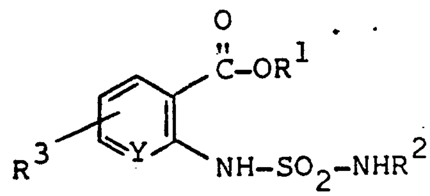

(I)

where
$R^1$ is hydrogen or $C_1$-$C_5$-alkyl,
$R^2$ is $C_1$-$C_5$-alkyl or $C_3$-$C_8$-cycloalkyl,
$R^3$ is hydrogen, $C_1$-$C_{10}$-alkyl or $C_1$-$C_4$-haloalkyl, and
Y is CH or N,
wherein a 2-aminobenzoic acid (nicotinic acid) derivative of the formula

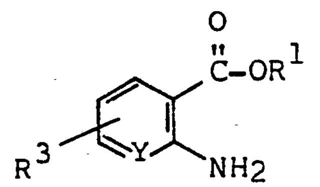

(II),

where
$R^1$, $R^3$ and Y have the above meanings,
a) is reacted with sulfur trioxide, chlorosulfonic acid or an adduct of sulfur trioxide with a tertiary amine in the presence of a tertiary amine A and of a diluent to give the corresponding sulfamic acid salt, and the latter is reacted with a primary amine of the formula
$R^2$-$NH_2$ (III),
where
$R^2$ has the above meanings.
in the presence of a phosphorus halides as dehydrating agent, or
b) is racted with a sulfamic acid of the formula
$R^2$-NH-$SO_3H$ (IV),
where $R^2$ has the above meanings, this acid, in the presence of a tertiary amine A, diluent and a phosphorus halide as dehydrating agent.

2. A process as claimed in claim 1, wherein a sulfamic acid salt of the formula
$R^2$-NH-$SO_3H$.A,
where $R^2$ and A have the meanings given in claim 1, is used.

3. A process as claimed in claim 1, wherein a sulfamic acid of the formula IV, or a salt thereof, is used which was obtained by reacting a primary amine of the formula III with sulfur trioxide or chlorosulfonic acid in the presence of a tertiary amine A and a diluent.

4. A process as claimed in claim 1, wherein the phosphorus halide is phosphorus oxychloride.

## Revendications

1. Procédé de préparation de N-phényl(pyridyle)-sulfonyldiamides de formule

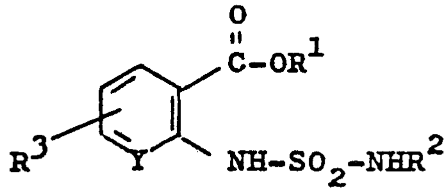

(I),

dans laquelle
$R^1$ représente hydrogène ou alkyle en $C_1$-$C_5$,
$R^2$ alkyle en $C_1$-$C_5$ ou cycloalkyle en $C_3$-$C_8$,

$R^3$ hydrogène, alkyle en $C_1$-$C_{10}$ ou halogénoalkyle en $C_1$-$C_4$, et
Y CH ou N.
caractérisé par le fait que l'on fait réagir un dérivé d'acide 2-amino-benzoique (nicotinique) de formule

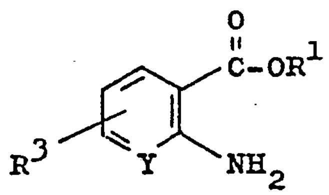

(II),

dans laquelle
$R^1$, $R^3$ et Y ont les significations sus-indiquées,
a) avec de l'anhydride sulfurique, de l'acide chlorosulfonique ou un produit d'addition d'anhydride sulfurique sur une amine tertiaire, en présence d'une amine tertaire A et d'un diluant, pour obtenir le sel d'acide sulfamidique correspondant, et celui-ci avec une amine primaire de formule
$R^2NH_2$ (III),
dans laquelle
$R^2$ a la signification sus-indiquée, en présence d'un halogénure de phosphore comme déshydratant, ou
b) avec un acide sulfamidique de formule
$R^2$-NH-$SO_3H$ (IV),
dans laquelle
$R^2$ a les significations sus-indiquées, ou un sel de cet acide, en présence d'une amine tertiaire A, un diluant et un halogénure de phosphore, comme déshydratant.

2. Procédé selon la revendication 1, caractérisé par le fait que l'on utilise un sel d'acide sulfamidique de formule
$R^2$-NH-$SO_3H$.A,
$R^2$ et A ayant les significations données dans la revendications 1.

3. Procédé selon la revendication 1, caractérisé par le fait que l'on utilise un acide sulfamidique de formule IV ou un sel de cet acide, qui a été obtenu par réaction d'une amine primaire de formule III avec de l'anhydride sulfurique ou de l'acide chlorosulfonique en présence d'une amine tertiaire A et d'un diluant.

4. Procédé selon la revendication 1, caractérisé par le fait que l'halogénure de phosphore est l'oxychlorure de phosphore.